# EUROPEAN PATENT SPECIFICATION

(11) **EP 4 531 666 B1**
(45) Date of publication and mention of the grant of the patent: **20.05.2026**
(21) Application number: 22732838.2
(22) Date of filing: 31.05.2022
(51) Int. Cl.: A61B 5/021, A61B 5/024, A61B 5/00

(54) **RADAR-BASED BLOOD PRESSURE MEASUREMENT**
RADARBASIERTE BLUTDRUCKMESSUNG
MESURE DE LA PRESSION ARTÉRIELLE BASÉE SUR UN RADAR

(43) Date of publication of application: 09.04.2025
(73) Proprietor: Google LLC, Mountain View, CA 94043 (US)
(72) Inventor: NONGPIUR, Rajeev, Mountain View, California 94043 (US); XU, Luzhou, Mountain View, California 94043 (US)
(74) Representative: Maikowski & Ninnemann Patentanwälte Partnerschaft mbB
(86) International application number: PCT/US2022/031531
(87) International publication number: WO 2023/234919

(56) References cited:
- DE-U1- 202016 105 262
- US-A1- 2016 345 845
- US-A1- 2017 296 093
- US-A1- 2019 298 208
- BUXI DILPREET ET AL: "Blood Pressure Estimation Using Pulse Transit Time From Bioimpedance and Continuous Wave Radar", IEEE TRANSACTIONS ON BIOMEDICAL ENGINEERING, IEEE, USA, vol. 64, no. 4, 1 April 2017 (2017-04-01), pages 917 - 927, XP011642873, ISSN: 0018-9294, [retrieved on 20170317], DOI: 10.1109/TBME.2016.2582472
- TSENG TZU-JUNG ET AL: "Noncontact Wrist Pulse Waveform Detection Using 24-GHz Continuous-Wave Radar Sensor for Blood Pressure Estimation", 2020 IEEE/MTT-S INTERNATIONAL MICROWAVE SYMPOSIUM (IMS), IEEE, 4 August 2020 (2020-08-04), pages 647 - 650, XP033839748, DOI: 10.1109/IMS30576.2020.9224111

## Description

### BACKGROUND

Blood pressure (BP) is an important vital statistic for measuring health. A person may desire to occasionally or periodically measure their BP to ensure it is within healthy limits. A sudden decrease or increase in BP can serve as a warning that a person should seek medical help. Typically, BP measurement devices for at home are inconvenient. Some require that a high level of pressure be applied by a cuff to the person's arm. Others require that a specialized device be clipped to a finger of a person. In still other arrangements, a hybrid approach is taken of combining an electrocardiogram (ECG) with a finger photoplethysmography (PPG) sensor, then deriving a pulse transit time (PTT), which can then be used to determine BP.

Such arrangements are not ideal, especially for a home environment. First, such arrangements require physical contact with a person. Second, a specialized device needs to be stored and retrieved each time the person desires to measure their BP. Embodiments detailed herein can address these and other issues. Relevant prior art documents are US 2016/345845 A1, US 2019/298208 A1 and US 2017/296093 A1.

### SUMMARY

In some embodiments, a method according to claim 1 for measuring blood pressure is presented.

Embodiments of such a method can include one or more of the following features: The extremity of the user can be one or more hands of the user. The extremity of the user can be one or more feet of the user. Analyzing the RF reflection signals at the first distance range and at the second distance range can be performed by one or more neural networks. The method can include determining a heart rate based on analyzing the RF reflection signals, wherein determining the blood pressure of the user is further based on the heart rate. The method can include determining a derived pulse waveform amplitude (DPWA) at the extremity of the user based on analyzing the RF reflection signals, wherein determining the blood pressure of the user is further based on the DPWA. The method can include receiving, by the processing system, an external blood pressure measurement made using a blood pressure device separate from a device comprising the radar sensor. The method can include comparing, by the processing system, the external blood pressure measurement and the determined blood pressure measurement. The method can include creating, by the processing system, a calibration profile for use in modifying a future determined blood pressure measurement. A machine learning model can be modified based on the calibration profile specific to the user. The radar sensor and the processing system can be integrated as part of a home assistant hub device that further comprises a display screen and speaker, wherein the indication of the determined blood pressure is output using the display screen, the speaker, or both.

In some embodiments, a blood pressure measurement system according to claim 8 is presented.

Embodiments of a system can include one or more of the following features: The system can include a housing, wherein the housing houses the radar subsystem and the processing system. The housing can house: a microphone, a speaker, and an electronic display, wherein the indication of the determined blood pressure is output via the electronic display. The extremity of the user can be one or more hands of the user. The extremity of the user can be one or more feet of the user. Analyzing the RF reflection signals at the first distance range and at the second distance range can be performed by one or more neural networks. The processing system can be further configured to determine a heart rate based on analyzing the RF reflection signals, wherein determining the blood pressure of the user is further based on the heart rate. The processing system can be further configured to determine a derived pulse waveform amplitude (DPWA) at the extremity of the user based on analyzing the RF reflection signals, wherein determining the blood pressure of the user is further based on the DPWA. The processing system can be configured to receive an external blood pressure measurement made using a blood pressure device separate from a device comprising the radar subsystem. The processing system can be configured to compare the external blood pressure measurement and the determined blood pressure measurement. The processing system can be configured to create a calibration profile for use in modifying a future determined blood pressure measurement. A machine learning model can be modified based on the calibration profile specific to the user.

In some embodiments, a non-transitory processor-readable medium according to claim 15 is presented.

### BRIEF DESCRIPTION OF THE DRAWINGS

A further understanding of the nature and advantages of various embodiments may be realized by reference to the following figures. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 illustrates an embodiment of a computerized device that can be used to perform radar-based BP measurements.
FIG. 2 illustrates a block diagram of an embodiment of a radar-based BP measurement system.
FIG. 3 illustrates a block diagram of a second embodiment of a radar-based BP measurement system.
FIG. 4 illustrates an embodiment of frequency-modulated continuous wave radar radio waves output by a radar subsystem.
FIG. 5 illustrates an embodiment of a smart home hub device that can be used to perform radar-based BP measurements.
FIG. 6 illustrates an exploded view of an embodiment of a smart home hub device that can be used to perform radar-based BP measurements.
FIG. 7 illustrates an embodiment of a user having the user's BP measured using a radar-based BP measurement system.
FIG. 8 illustrates another embodiment of a user having the user's BP measured using a radar-based BP measurement system.
FIG. 9 illustrates an embodiment of a method for measuring BP.
FIG. 10 illustrates another embodiment of a method for measuring BP.
FIG. 11 illustrates an embodiment of a method for calibrating a radar-based BP measurement system.

### DETAILED DESCRIPTION

Pulse transmit time (PTT) is indicative of the amount of time it takes a pulse pressure wave (PPW) to travel from a person's aortic value to a person's extremities, such as hands or feet. As blood pressure of a person increases, the PTT decreases. Therefore, by determining a PTT, possibly in conjunction with other measurements, BP can be determined.

Embodiments detailed herein are focused on embodiments of a radar-based BP measurement system and associated methods that allow for BP measurement solely using radar and without requiring physical contact with a user. A radar-based BP measurement system can include a radar sensor that can detect small movements at discrete distances. Therefore, movement a farther distance from the radar sensor (e.g., movement of a PPW at an aortic valve) can be distinguished from movement at a closer distance to the radar sensor (e.g., movement of a PPW in a user's hands or feet). The PTT can be calculated using such radar-based measurements by determining the amount of time that elapsed between detecting the PPW at the aortic valve and the PPW at one or more extremities of the user.

As detailed herein, additional measurements can be performed to determine a more accurate BP of a user, such as PPW magnitude, and heart rate (HR). In some embodiments, as detailed herein, a trained machine learning model is used to either partially or fully analyze data obtained from a radar sensor.

By virtue of using only radar measurements to determine the PTT (and possibly the HR and/or PPW magnitude), the BP of a user can be measured without physical contact with the user. Such a BP measurement system can be incorporated as part of a device that includes a radar sensor, such as a home assistant device. A home assistant device can present information to the user about their measured BP and may be in a physical position that lends itself to easy BP measurement. Use of such a form of device can be highly beneficial since it may typically be displayed in a prominent position within a person's home, such as on a table or bedstand, and can provide an easy opportunity for a user to conduct a BP measurement.

BP measurements, as detailed herein, can only be performed with explicit approval of the user. The user may need to remain in a specific physical position and be relatively motionless in order for the BP measurement to be performed. During such a time, visual and/or auditory messages can be output indicating that the BP of the user is being measured. Further, the user may be required to provide a consent allowing the user's BP to be measured. The user may retain the ability to delete any BP measurement made using such a system.

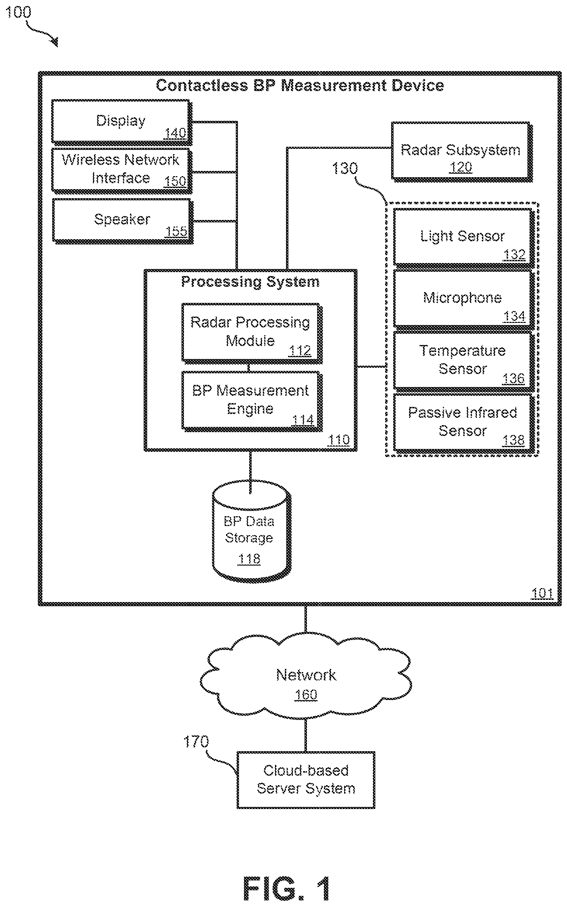

Further detail of these and other embodiments is provided in relation to the figures. FIG. 1 illustrates an embodiment of BP measurement system 100 ("system 100") that can be used to performed radar-based BP measurements. System 100 includes a contactless BP measurement device 101 ("device 101"); and can include network 160; and cloud-based server system 170. Device 101 may generally be a smart home assistant device, a smartphone, a smartwatch, laptop, gaming device, or a smart home hub device that is used to interact with various smart home devices present within a home. Device 101 can include: processing system 110; BP data storage 118; radar subsystem 120; environmental sensor suite 130; display 140; wireless network interface 150; and speaker 155. Generally, device 101 can include a housing that houses all of the components of device 101.

Processing system 110 can include one or more processors configured to perform various functions, such as the functions of: radar processing module 112; and BP measurement engine 114. Processing system 110 can include one or more special-purpose or general-purpose processors. Such special-purpose processors may include processors that are specifically designed to perform the functions detailed herein. Such special-purpose processors may be ASICs or FPGAs which are general-purpose components that are physically and electrically configured to perform the functions detailed herein. Such general-purpose processors may execute special-purpose software that is stored using one or more non-transitory processor-readable mediums, such as random access memory (RAM), flash memory, a hard disk drive (HDD), or a solid state drive (SSD).

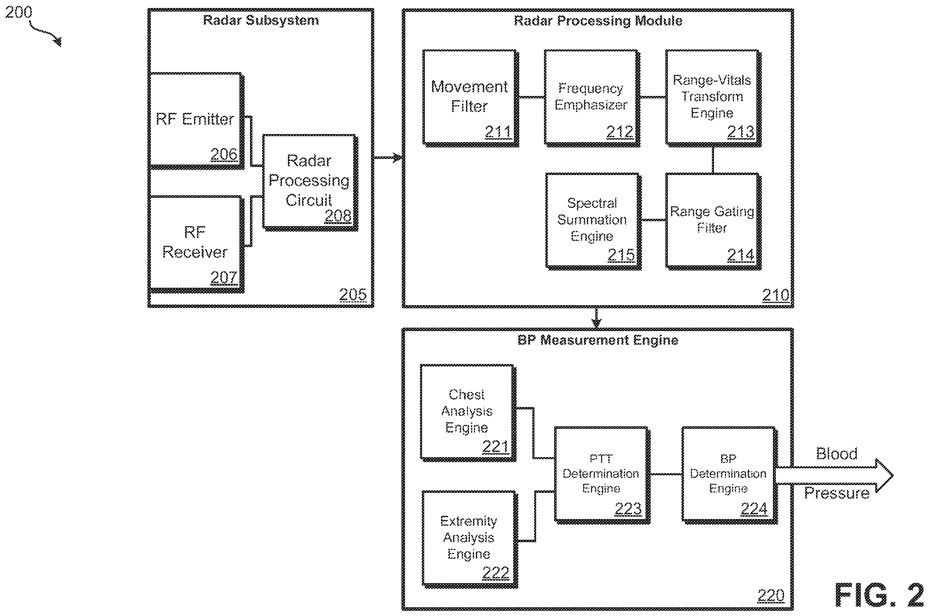

Radar subsystem 120 (also referred to as a radar sensor) can be a single integrated circuit (IC) that emits, receives, and outputs data indicative of a received, reflected waveform. The output of radar subsystem 120 may be analyzed using radar processing module 112 of processing system 110. Further detail regarding radar subsystem 120 and radar processing module 112 is provided in relation to **FIGS.** 2-4.

Device 101 may include one or more environmental sensors, such as all, one, or some combination of the environmental sensors provided as part of environmental sensor suite 130. Environmental sensor suite 130 can include: light sensor 132; microphone 134; temperature sensor 136; and passive infrared (PIR) sensor 138. In some embodiments, multiple instances of some or all of these sensors may be present. For instance, in some embodiments, multiple microphones may be present. Light sensor 132 may be used for measuring an ambient amount of light present in the general environment of device 101. Microphone 134 may be used for measuring an ambient noise level present in the general environment of device 101. Temperature sensor 136 may be used for measuring an ambient temperature of the general environment of device 101. PIR sensor 138 may be used to detect moving living objects (e.g., persons, pets) within the general environment of device 101. Other types of environmental sensors are possible. For instance, a camera and/or humidity sensor may be incorporated as part of environmental sensor suite 130. As another example, active infrared sensors may be included. In some embodiments, some data, such as humidity data, may be obtained from a nearby weather station that has data available via the Internet. In some embodiments, active acoustic sensing methods, including, but not limited to, sonar and ultrasound, and including either single or arrayed acoustic sources and/or receivers may be implemented. Such arrangements may be used as one or more adjunct sensing modalities incorporated with the other sensors and methods described herein.

In some embodiments, one, some, or all of sensors of environmental sensor suite 130 may be external device to 101. For instance, one or more remote environmental sensors may communicate with device 101, either directly (e.g., via a direct wireless communication method, via a low-power mesh network) or indirectly (e.g., through one or more other devices via the low-power mesh network, via an access point of a network, via a remote server).

Device 101 may include various interfaces. Display 140 can allow processing system 110 to present information for viewing by one or more users. Wireless network interface 150 can allow for communication using a wireless local area network (WLAN), such as a WiFi-based network. Speaker 155 can allow for sound, such as synthesized speech, to be output. For instance, responses to spoken commands received via microphone 134 may be output via speaker 155 and/or display 140. The spoken commands may be analyzed locally by device 101 or may be transmitted via wireless network interface 150 to cloud-based server system 170 for analysis. A response, based on the analysis of the spoken command, can be sent back to device 101 via wireless network interface 150 for output via speaker 155 and/or display 140. Additionally or alternatively, the speaker 155 and microphone 134 may be collectively configured for active acoustic sensing, including ultrasonic acoustic sensing. Additionally or alternatively, other forms of wireless communication may be possible, such as using a low-power wireless mesh network radio and protocol (e.g., Thread) to communicate with various smart home devices. In some embodiments, a wired network interface, such as an Ethernet connection, may be used for communication with a network. Further, the evolution of wireless communication to fifth generation (5G) and sixth generation (6G) standards and technologies provides greater throughput with lower latency which enhances mobile broadband services. 5G and 6G technologies also provide new classes of services, over control and data channels, for vehicular networking (V2X), fixed wireless broadband, and the Internet of Things (IoT). Such standards and technologies may be used for communication by device 101.

A low-power wireless mesh network radio and protocol may be used for communicating with power limited devices. A power-limited device may be an exclusively battery powered device. Such devices may rely exclusively on one or more batteries for power and therefore, the amount of power used for communications may be kept low in order to decrease the frequency at which the one or more batteries need to be replaced. In some embodiments, a power-limited device may have the ability to communicate via a relatively high power network (e.g., WiFi) and the low-power mesh network. The power-limited device may infrequently use the relatively high power network to conserve power. Examples of such power-limited devices include environmental sensors (e.g., temperature sensors, carbon monoxide sensors, smoke sensors, motion sensors, presence detectors) and other forms of remote sensors.

Notably, some embodiments of device 101 do not have any still camera or video camera. By not incorporating an on-board camera, users nearby may be reassured about their privacy. For example, device 101 can typically be installed in a user's bedroom. For many reasons, a user would not want a camera located in such a private space or aimed toward the user while the user is sleeping or performing some other private activity. In other embodiments, device 101 may have a camera, but the camera's lens may be obscured by a mechanical lens shutter. In order to use the camera, the user may be required to physically open the shutter to allow the camera to have a view of the environment of device 101. The user can be assured of privacy from the camera when the shutter is closed.

Wireless network interface 150 can allow for wireless communication with network 160. Network 160 can include one or more public and/or private networks. Network 160 can include a local wired or wireless network that is private, such as a home wireless local area network. Network 160 may also include a public network, such as the Internet. Network 160 can allow for device 101 to communicate with remotely located cloud-based server system 170.

Cloud-based server system 170 can provide device 101 with various services. Regarding BP data, cloud-based server system 170 can include processing and storage services for BP-related data. While the embodiment of FIG. 1 involves processing system 110 performing BP measurement, in other embodiments, such functions may be performed by cloud-based server system 170. Also, in addition or in alternate to BP data storage 118 being used to store BP data, BP-related data may be stored by cloud-based server system 170, such as mapped to a common user account to which device 101 is linked. If multiple users are monitored, the BP data may be stored and mapped to a master user account or to the corresponding users' accounts.

Regardless of whether a single user or multiple users use device 101 to measure BP, each user may be required to provide their informed consent. Such informed consent may involve each user consenting to an end user agreement that involves data being used in compliance with HIPAA and/or other generally accepted security and privacy standards for health information. Periodically, user may be required to renew their consent to collection of BP data, such as annually. In some embodiments, each end user may receive a periodic notification, such as via a mobile device (e.g., smartphone) that reminds each user that their BP data is being collected and analyzed and offers each user the option to disable such data collection.

Cloud-based server system 170 may additionally or alternatively provide other cloud-based services. For instance, device 101 may additionally function as a home assistant device. A home assistant device may respond to vocal queries from a user. In response to detecting a vocal trigger phrase being spoken, device 101 may record audio via microphone 134. A stream of the audio may be transmitted to cloud-based server system 170 for analysis. Cloud-based server system 170 may perform a speech recognition process, use a natural language processing engine to understand the query from the user, and provide a response to be output by device 101 as synthesized speech, an output to be presented on display 140, and/or a command to be executed by device 101 (e.g., raise the volume of device 101) or sent to some other smart home device. Further, queries or commands may be submitted to cloud-based server system 170 via display 140, which may be a touchscreen. For instance, device 101 may be used to control various smart home devices or home automation devices. Such commands may be sent directly by device 101 to the device to be controlled or may be sent via cloud-based server system 170.

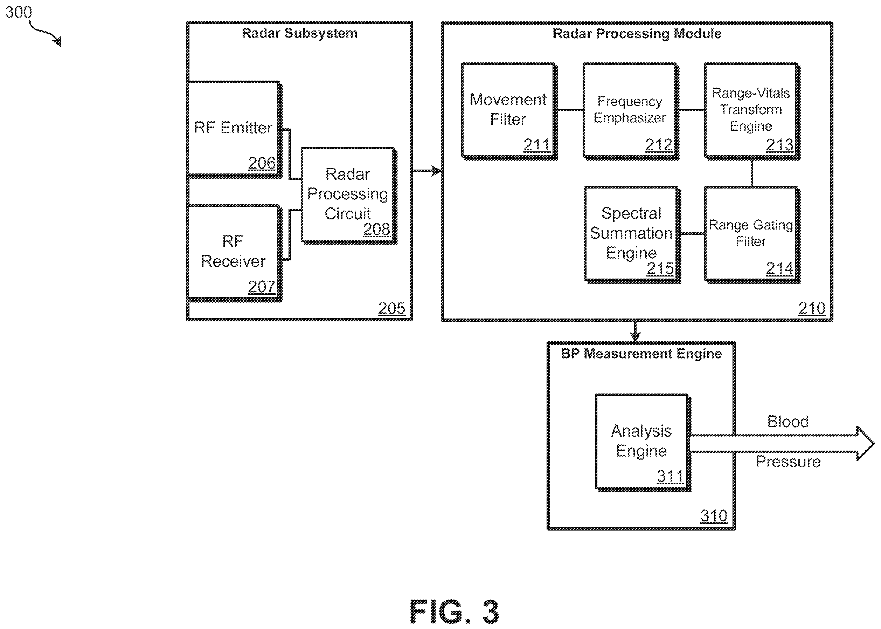

Based on data output by radar processing module 112, BP measurement engine 114 may be used to determine a BP of a user. Further detail regarding the components of BP measurement engine 114 are provided in relation to FIGS. 2 and 3.

FIG. 2 illustrates a block diagram of an embodiment of a radar-based BP measurement system 200 ("system 200"). System 200 can include radar subsystem 205 (which can represent an embodiment of radar subsystem 120); radar processing module 210 (which can represent an embodiment of radar processing module 112); and BP measurement engine 220.

Radar subsystem 205 may include RF emitter 206, RF receiver 207, and radar processing circuit 208. RF emitter 206 can emit radio waves, such as in the form of continuous-wave (CW) radar. RF emitter 206 may use frequency-modulated continuous-wave (FMCW) radar. The FMCW radar may operate in a burst mode or continuous sparse-sampling mode. In burst mode, a frame or burst of multiple chirps, with the chirps spaced by a relatively short period of time, may be output by RF emitter 206. Each frame may be followed by a relatively long amount of time until a subsequent frame. In a continuous sparse-sampling mode, frames or bursts of chirps are not output, rather chirps are output periodically. The spacing of chirps in the continuous sparse sampling mode may be greater in duration than the spacing between chirps within a frame of the burst mode. In some embodiments, radar subsystem 205 may operate in a burst mode, but output raw chirp waterfall data for each burst may be combined (e.g., averaged) together to create simulated continuous sparse-sampled chirp waterfall data. In some embodiments, raw waterfall data gathered in burst mode may be preferable for gesture detection while raw waterfall data gathered in a continuously sparse sampling mode may be preferable for certain functions, such as BP measurement, sleep tracking, vital sign detection, and, generally, health monitoring. Gesture detection may be performed by other hardware or software components that use the output of radar subsystem 205 that are not illustrated.

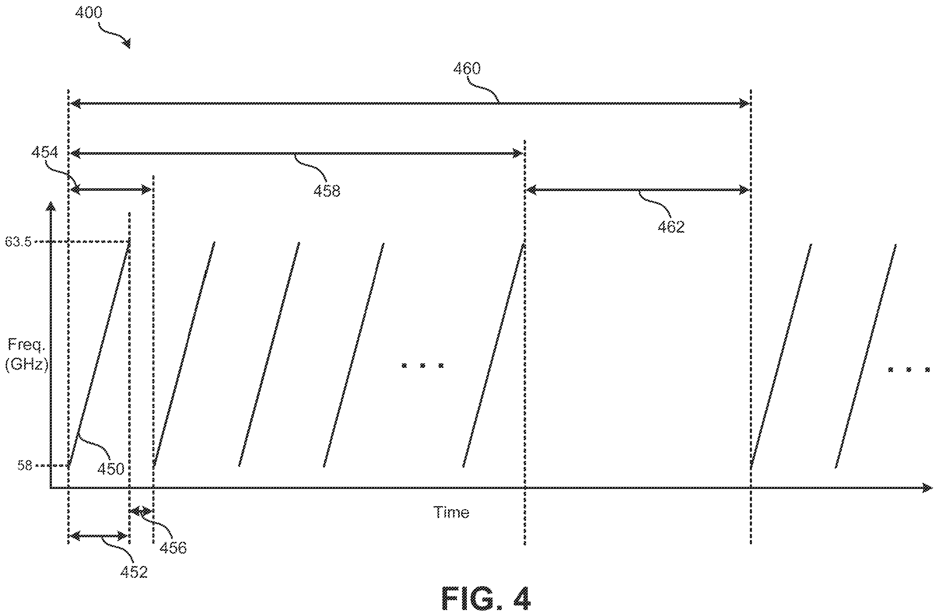

RF emitter 206 may include one or more antennas and may transmit at or about 60 GHz. The frequency of radio waves transmitted may repeatedly sweep from a low to high frequency (or the reverse). The power level used for transmission may be very low such that radar subsystem 205 has an effective range of several meters or an even shorter distance. Further detail regarding the radio waves generated and emitted by radar subsystem 205 are provided in relation to FIG. 4.

RF receiver 207 includes one or more antennas, distinct from the transmit antenna(s), and may receive radio wave reflections off of nearby objects of radio waves emitted by RF emitter 206. The reflected radio waves may be interpreted by radar processing circuit 208 by mixing the radio waves being transmitted with the reflected received radio waves, thereby producing a mixed signal that can be analyzed for distance. Based on this mixed signal, radar processing circuit 208 may output raw waveform data, which can also be referred to as the waterfall data for analysis by a separate processing entity. Radar subsystem 205 may be implemented as a single integrated circuit (IC) or radar processing circuit 208 may be a separate component from RF emitter 206 and RF receiver 207. In some embodiments, radar subsystem 205 is integrated as part of device 101 such that RF emitter 206 and RF receiver 207 are pointing in a same direction as display 140. In other embodiments, an external device that includes radar subsystem 205 may be connected with device 101 via wired or wireless communication. For example, radar subsystem 205 may be an add-on device to a home assistant device.

Raw waveform data may be passed from radar subsystem 205 to radar processing module 210. The raw waveform data passed to radar processing module 210 may include waveform data indicative of continuous sparse reflected chirps due to radar subsystem 205 operating in a continuous sparse sampling mode or due to radar subsystem 205 operating in a burst mode and a conversion process to simulate raw waveform data produced by radar subsystem 205 operating in a continuous sparse sampling mode being performed. Processing may be performed to convert burst sampled waveform data to continuous sparse samples using an averaging process, such as each reflected group of burst radio waves being represented by a single averaged sample. Radar processing module 210 may include one or more processors. Radar processing module 210 may include one or more special-purpose or general-purpose processors. Special-purpose processors may include processors that are specifically designed to perform the functions detailed herein. Such special-purpose processors may be ASICs or FPGAs which are general-purpose components that are physically and electrically configured to perform the functions detailed herein. General-purpose processors may execute special-purpose software that is stored using one or more non-transitory processor-readable mediums, such as random access memory (RAM), flash memory, a hard disk drive (HDD), or a solid state drive (SSD). Radar processing module 210 may include: movement filter 211; frequency emphasizer 212; range-vitals transform engine 213; range gating filter 214; and spectral summation engine 215. Each of the components of radar processing module 210 may be implemented using software, firmware, or as specialized hardware.

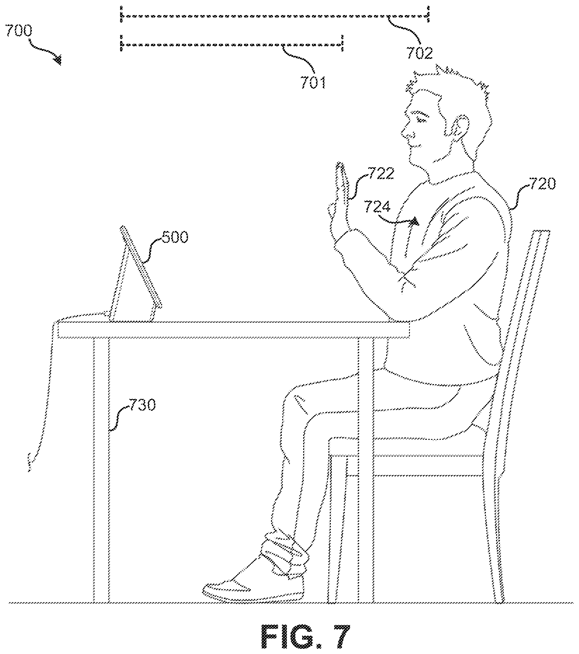

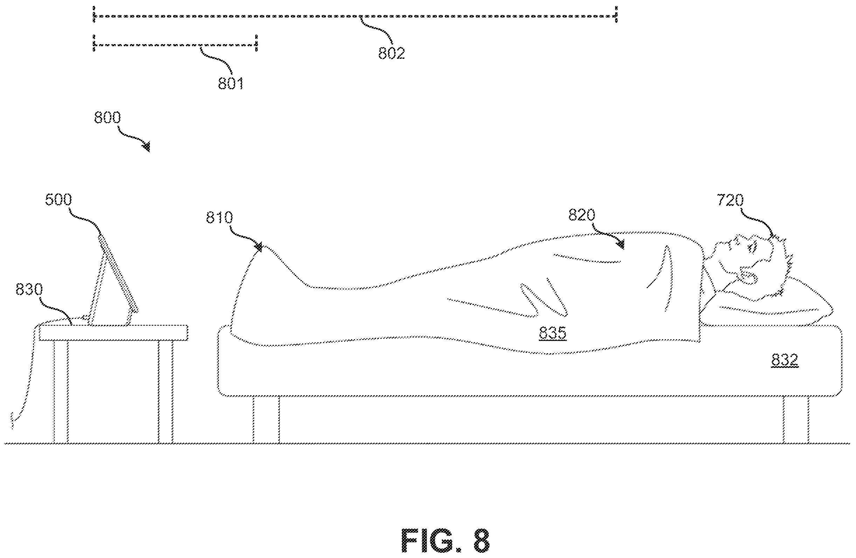

The raw waveform data output by radar subsystem 205 may be received by radar processing module 210 and first processed using movement filter 211. In some embodiments, it is important that movement filter 211 is the initial component used to perform filtering. That is, the processing performed by radar processing module 210 is not commutative in some embodiments. Typically, vital sign determination (which includes BP measurement) may occur when a monitored user is in a low movement environment, such as detailed in relation to FIGS. 7 and 8. In such an environment, there may typically be little movement. For what movement is present, the movement may be attributed to the user's vital signs, including movement due to breathing and movement due to the monitored user's heartbeat. In such an environment, a large portion of emitted radio waves from RF emitter 206 may be reflected by static objects in the vicinity of the monitored user, such as furniture, bedding, walls, etc. Therefore, a large portion of the raw waveform data received from radar subsystem 205 may be unrelated to user movements and the user's vital measurements.

Movement filter 211 may include a waveform buffer that buffers "chirps" or slices of received raw waveform data. For instance, sampling may occur at a rate of 10 Hz. In other embodiments, sampling may be slower or faster. Movement filter 211 may buffer twenty seconds of received raw waveform chirps in certain embodiments. In other embodiments, a shorter or longer duration of buffered raw waveform data is buffered. This buffered raw waveform data can be filtered to remove raw waveform data indicative of stationary objects. That is, for objects that are moving, such as a monitored user's chest, the user's heartbeat and breathing rate will affect the distance and velocity measurements made by radar subsystem 205 and output to movement filter 211. This movement of the user will result in "jitter" in the received raw waveform data over the buffered time period. More specifically, jitter refers to the phase shifts caused by moving objects reflecting emitted radio waves. Rather than using the reflected FMCW radio waves to determine a velocity of the moving objects, the phase shift induced by the motion in the reflected radio waves can be used to measure vital statistics, including heartrate and breathing rate, as detailed herein.

For stationary objects, such as furniture, a zero phase shift (i.e., no jitter) will be present in the raw waveform data over the buffered time period. Movement filter 211 can subtract out such raw waveform data corresponding to stationary objects such that motion-indicative raw waveform data is passed to frequency emphasizer 212 for further analysis. Raw waveform data corresponding to stationary objects may be discarded or otherwise ignored for the remainder of processing by radar processing module 210.

In some embodiments, an infinite impulse response (IIR) filter is incorporated as part of movement filter 211. Specifically, a single-pole IIR filter may be implemented to filter out raw waveform data that is not indicative of movement. Therefore, the single-pole IIR filter may be implemented as a high-pass, low-block filter that prevents raw waveform data indicative of movement below a particular frequency from passing through to frequency emphasizer 212. The cut-off frequency may be set based on known limits to human vital signs. For example, a breathing rate may be expected to be between 10 and 60 breaths per minute. Movement data indicative of a lower frequency than 10 breaths per minute may be excluded by the filter. In some embodiments, a band-pass filter may be implemented to exclude raw waveform data indicative of movement at high frequencies that are impossible or improbable for human vital signs. For instance, a heartrate, which can be expected to be above a breathing rate, may be unlikely to be above 150 beats per minute for a person in a resting or near-resting state. Raw waveform data indicative of a higher frequency may be filtered out by the band pass filter.

The vital signs of the monitored user being measured are periodic impulse events: a user's heartrate may vary over time, but it can be expected that the user's heart will continue to beat periodically. This beating is not a sinusoidal function, but rather may be understood as an impulse event, more analogous to a square wave having a relatively low duty cycle that induces motion in the user's body. Frequency components due to this spectral leakage should be deemphasized.

Frequency emphasizer 212 may work in conjunction with range-vitals transform engine 213 to determine the frequency components of the raw waveform data attributable to vital statistics, such as a pulse of a user. Frequency emphasizer 212 may use frequency windowing, such as a 2D Hamming window (other forms of windowing are possible, such as a Hann window), to emphasize important frequency components of the raw waveform data and to deemphasize or remove waveform data that is attributable to spectral leakage outside of the defined frequency window. Such frequency windowing may decrease the magnitude of raw waveform data that is likely due to processing artifacts. The use of frequency windowing can help reduce the effects of data-dependent processing artifacts while preserving data relevant for being able to separately determine heartrate and breathing rate.

For a stationary tabletop or bedside FMCW radar-based monitoring device, which may be positioned within 1 to 2 meters of the one or more users being monitored to detect heartrate, a 2D Hamming window that emphasizes frequencies in the range of 30 to 150 bpm (0.5 to 2.5 Hz) for heartbeat provides for sufficiently good signals to make reliable measurements without requiring advance knowledge of the subject's age or medical history.

Since heartrate and breathing rate are periodic impulse events, the frequency domain heartrate, and breathing rate may be represented by different fundamental frequencies, but each may have many harmonic components at higher frequencies. One of the primary purposes of frequency emphasizer 212 may be to prevent the frequency ripples of harmonics of the monitored user's heartrate from affecting any other vital statistic, such as breathing rate, being measured. While frequency emphasizer 212 may use a 2D Hamming window, it should be understood that other windowing functions or isolating functions can be used to help isolate frequency ripples of the monitored user's breathing rate from the frequency ripples of the monitored user's heartrate.

Range-vitals transform engine 213 analyzes the received motion-filtered waveform data to identify and quantify the magnitude of movement at specific frequencies. More particularly, range-vitals transform engine 213 analyzes phase jitter over time to detect relatively small movements due to a user's vital signs that have a relatively low frequency, such as breathing rate and heart rate. The analysis of range-vitals transform engine 213 may assume that the frequency components of the motion waveform data are sinusoidal. Further, the transform used by range-vitals transform engine 213 can also identify the distance at which the frequency is observed. Frequency, magnitude, and distance can all be determined at least in part because radar subsystem 205 uses an FMCW radar system.

Range-vitals transform engine 213 can perform a series of Fourier transform (FT) to determine the frequency components of the received raw waveform data output by frequency emphasizer 212. Specifically, a series of fast Fourier transform (FFT) may be performed by range-vitals transform engine 213 to determine the specific frequencies and magnitudes of waveform data at such frequencies.

Waveform data obtained over a period of time can be expressed in multiple dimensions. A first dimension (e.g., along the y-axis) can relate to multiple samples of waveform data from a particular chirp and a second dimension (e.g., along the x-axis) relates to a particular sample index of waveform data gathered across multiple chirps. A third dimension of data (e.g., along the z-axis) is present indicative of the intensity of the waveform data.

Multiple FFTs may be performed based on the first and second dimension of the waveform data. FFTs may be performed along each of the first and second dimensions: an FFT may be performed for each chirp and an FFT may be performed for each particular sample index across multiple chirps that occurred during the period of time. An FFT performed on waveform data for a particular reflected chirp can indicate one or more frequencies, which, in FMCW radar, are indicative of the distances at which objects are present that reflected emitted radio waves. An FFT performed for a particular sample index across multiple chirps can measure the frequency of phase jitter across the multiple chirps. Therefore, the FFT of the first dimension can provide the distance at which a vital statistic is present and the FFT of the second dimension can provide a frequency of the vital statistic. The output of the FFTs performed across the two dimensions is indicative of: 1) the frequencies of vital statistics; 2) the ranges at which the vital statistics were measured; and 3) the magnitudes of the measured frequencies. In addition to values due to vital statistics being present in the data, noise may be present that is filtered, such as using spectral summation engine 215. The noise may be partially due to heartrate and breathing not being perfect sinusoidal waves.

To be clear, the transform performed by range-vitals transform engine 213 differs from a range-Doppler transform. Rather than analyzing changes in velocity (as in a range-Doppler transform), periodic changes in phase shift over time are analyzed as part of the range-vitals transform. The range-vitals transform is tuned to identify small movements (e.g., breathing, expansion of blood vessels and valves) occurring over a relatively long period of time by tracking changes in phase, referred to as phase jitter.

Range gating filter 214 is used to monitor a defined range of interest and exclude waveform data due to movement beyond the defined range of interest. For arrangements detailed herein, the defined range of interest may be 0 to 1 meter. In some embodiments, this defined range of interest may be different or possibly set by a user (e.g., via a training or setup process) or by a service provider. In some embodiments, a goal of this arrangement may be to monitor the one person closest to the device (and exclude or segregate data for any other person farther away, such as a person sleeping next to the person being monitored). Therefore, range-vitals transform engine 213 and range gating filter 214 serve to segregate, exclude, or remove movement data attributed to objects outside of the defined range of interest and sum the energy of movement data attributed to objects within the defined range of interest. The output of range gating filter 214 may include data that has a determined range within the permissible range of range gating filter 214. The data may further have a frequency dimension and a magnitude. Therefore, the data may possess three dimensions.

As part of range-vitals transform engine 213 and/or range gating filter 214, data gathered at particular distances may be binned together. Some number of range bins is created, such as 256. Motion that is detected within a particular distance range corresponding to a bin is grouped together for analysis. As will be detailed herein, a first distance bin is identified at which a user's pulse is detected at or near the user's aortic valve; a second distance bin is identified at which a user's pulse is detected at a user's extremity (e.g., hands, feet).

Spectral summation engine 215 may receive the output from range gating filter 214. Spectral summation engine 215 may function to transfer the measured energy of harmonic frequencies of the user's heartrate and sum the harmonic frequency energy onto the fundamental frequency's energy for each distance bin. This function can be referred to as a harmonic sum spectrum (HSS). As previously noted, vital statistics such as heartrate and breathing rate are not sinusoidal; therefore, in the frequency domain, harmonics will be present at frequencies higher than the fundamental frequency of the user's breathing rate and the fundamental frequency of the user's heartrate. One of the primary purposes of spectral summation engine 215 can be to prevent harmonics of the monitored user's breathing rate from affecting the frequency measurement of the monitored user's heartrate (and the reverse). The HSS may be performed at the second order by summing the original spectrum with a down-sampled instance (by a factor of two) of the spectrum. This process may also be applied at higher order harmonics such that their respective spectra are added to the spectrum at the fundamental frequency. The HSS may be applied for some or all distance bins in which a pulse (and/or breathing) is detected.

The output of radar processing module 210 can be passed to BP measurement engine 220. BP measurement engine 220, which may be implemented using the same or a different processing system as radar processing module 210, can include: chest analysis engine 221, extremity analysis engine 222, PTT determination engine 223; and BP determination engine 224.

Chest analysis engine 221 can serve to detect a pulse waveform due to the user's heartbeat at the user's chest. More specifically, chest analysis engine 221 may detect the pulse waveform at or near the aortic value of the user. Chest analysis engine 221 may analyze the range binned data received from spectral summation engine 215. Since a heartbeat causes a large amount of movement at and around an aortic value, a distance bin having a greatest amount of movement attributed to the pulse waveform can be the distance bin selected and analyzed by chest analysis engine 221. Chest analysis engine 221 can be a trained machine learning model, such as a trained neural network, that can identify a heartbeat of the user and can assign a time to the heartbeat.

Extremity analysis engine 222 can serve to detect a pulse waveform due to the user's pulse at an extremity of the user, such as the user's hand(s) or foot (or feet). Extremity analysis engine 222 may analyze the range binned data received from spectral summation engine 215. In some embodiments, a user is requested to set their hands or feet a defined distance from radar subsystem 120, so the distance bin may be known from this request. In other embodiments, based on a position in which the user is requested to hold their hands or feet, such as detailed in relation to FIGS. 7 and 8, the distance bin determined for the user's extremity may be the closest distance bin to radar subsystem 120 at which the pulse can be detected. Extremity analysis engine 222 can be a trained machine learning model, such as a trained neural network, that can identify a pulse of the user in the user's extremity and can assign a time to the heartbeat.

In some embodiments, rather than using a trained machine learning mode as part of chest analysis engine 221 and extremity analysis engine 222, an algorithm can be used to time tag a pulse waveform at the extremity and chest.

PTT determination engine 223 can determine at least the PTT by calculating a difference in time between the pulse waveform measured at the user's chest by chest analysis engine 221 and the pulse waveform (attributed to the same heartbeat) measured at the user's extremity by extremity analysis engine 222. In some embodiments, additional data based on the pulse waveform measured by chest analysis engine 221 and/or extremity analysis engine 222 can be determined. This data can include a derived pulse waveform amplitude (DPWA), which is indicative of the magnitude of the pulse as measured at the user's extremity and/or chest. In some embodiments, separate DPWA values are calculated for the extremity and chest. This data can additionally or alternatively include heartrate (HR) data by determining a number of pulse waveforms over a period of time. In some embodiments, separate HR values are calculated for the extremity and chest. PTT, HR, DPWA, or some combination thereof may be output to BP determination engine 224.

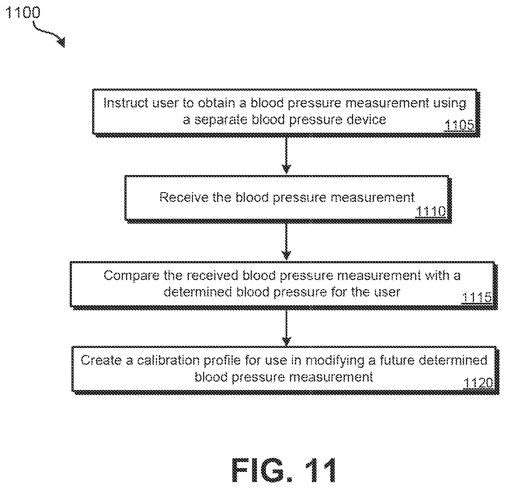

BP determination engine 224 may be an algorithm or look-up arrangement that uses PTT, HR, and/or DPWA to calculate a BP, which can include systolic and diastolic blood pressures. In some embodiments, a trained machine learning model can receive these three values as an input and can output systolic and diastolic blood pressures. In some embodiments, a calibration profile is applied to adjust the determined BP based on one or more measurements of the user's BP taken via another form of BP measurement device, such as detailed in relation to **FIG. 11****.** The output of BP determination engine 224 may be output for presentation via display 140, speaker 155, and/or stored by BP data storage 118 and/or cloud-based server system 170.

**FIG. 3** illustrates a block diagram of an embodiment of a radar-based BP measurement system 300 ("system 300"). System 200 can include radar subsystem 205 (which can represent an embodiment of radar subsystem 120); radar processing module 210 (which can represent an embodiment of radar processing module 112); and BP measurement engine 220. System 300 may function similarly to system 200 with the exception of BP measurement engine 310. BP measurement engine 310 may include only analysis engine 311. Analysis engine 311 can be a trained machine learning model, such as a neural network, that receives the output of radar processing module 210 as its input. Analysis engine 311 may be trained to output BP measurements, such as systolic and diastolic values, by directly analyzing the output of radar processing module 210.

To train analysis engine 311, a large number of measurements of users may be made using a system similar to radar processing module 210. The output data may be mapped to a blood pressure of the users gathered using an accurate BP measurement device. This training data may then be used to construct a machine learning model that can determine blood pressure based on the output of radar processing module 210.

In some embodiments, a calibration profile is applied to adjust the determined BP based on one or more measurements of the user's BP taken via another form of BP measurement device, such as detailed in relation to FIG. 11. In system 300, a layer of analysis engine 311 can be created or altered to account for the user's calibration profile.

**FIG. 4** illustrates an embodiment of chirp timing diagram 400 for frequency modulated continuous wave (FMCW) radar radio waves output by a radar subsystem. Chirp timing diagram 400 is not to scale. Radar subsystem 405 may generally output radar in the pattern of chirp timing diagram 400. Chirp 450 represents a continuous pulse of radio waves that sweeps up in frequency from a low frequency to a high frequency. In other embodiments, individual chirps may continuously sweep down from a high frequency to a low frequency, from a low frequency to a high frequency, and back to a low frequency, or from a high frequency to a low frequency and back to a high frequency. In some embodiments, the low frequency is 58 GHz and the high frequency is 63.5 GHz. (For such frequencies, the radio waves may be referred to as millimeter waves.) In some embodiments, the frequencies are between 57 and 64 GHz. The low frequency and the high frequency may be varied by embodiment. For instance, the low frequency and the high frequency may be between 45 GHz and 80 GHz. The frequencies may be selected at least in part to comply with governmental regulation. In some embodiments, each chirp includes a linear sweep from a low frequency to a high frequency (or the reverse). In other embodiments, an exponential or some other pattern may be used to sweep the frequency from low to high or high to low.

Chirp 450, which can be representative of all chirps in chirp timing diagram 400, may have chirp duration 452 of 128 µs. In other embodiments, chirp duration 452 may be longer or shorter, such as between 50 µs and 1 ms. In some embodiments, a period of time may elapse before a subsequent chirp is emitted. Inter-chirp pause 456 may be 405.33 µs. In other embodiments, inter-chirp pause 456 may be longer or shorter, such as between 10 µs and 1 ms. In the illustrated embodiment, chirp period 454, which includes chirp 450 and inter-chirp pause 456, may be 333.33 µs. This duration varies based on the selected chirp duration 452 and inter-chirp pause 456.

A number of chirps that are output, separated by inter-chirp pauses, may be referred to as frame 458 or frame 458. Frame 458 may include twenty chirps. In other embodiments, the number of chirps in frame 458 may be greater or fewer, such as between 1 and 100. The number of chirps present within frame 458 may be determined based upon a maximum amount of power that is desired to be output within a given period of time. The FCC or other regulatory agency may set a maximum amount of power that is permissible to be radiated into an environment. For example, a duty cycle requirement may be present that limits the duty cycle to less than 10% for any 33 ms time period. In one particular example in which there are twenty chirps per frame, each chirp can have a duration of 128 us, and each frame can be 33.33 ms in duration. The corresponding duty cycle is (20 frames)*(. 128 ms)/(33.33 ms), which is about 7.8%. By limiting the number of chirps within frame 458 prior to an inter-frame pause, the total amount of power output may be limited. In some embodiments, the peak EIRP (effective isotropically radiated power) may be 13 dBm (20 mW) or less, such as 12.86 dBm (19.05 mW). In other embodiments, the peak EIRP is 15 dBm or less and the duty cycle is 15% or less. In some embodiments, the peak EIRP is 40 dBm or less. That is, at any given time, the amount of power radiated by the radar subsystem might never exceed such values. Further, the total power radiated over a period of time may be limited.

Frames may be transmitted at a frequency of 30 Hz (33.33 ms) as shown by time period 460. In other embodiments, the frequency may be higher or lower. The frame frequency may be dependent on the number of chirps within a frame and the duration of inter-frame pause 462. For instance, the frequency may be between 1 Hz and 50 Hz. In some embodiments, chirps may be transmitted continuously, such that the radar subsystem outputs a continuous stream of chirps interspersed with inter-chirp pauses. Tradeoffs can be made to save on the average power consumed by the device due to transmitting chirps and processing received reflections of chirps. Inter-frame pause 462 represents a period of time when no chirps are output. In some embodiments, inter-frame pause 462 is significantly longer than the duration of frame 458. For example, frame 458 may be 6.66 ms in duration (with chirp period 454 being 333.33 µs and 40 chirps per frame). If 33.33 ms occur between frames, inter-frame pause 462 may be 46.66 ms. In other embodiments, the duration of inter-frame pause 462 may be larger or smaller, such as between 15 ms and 40 ms.

In the illustrated embodiment of **FIG. 4****,** a single frame 458 and the start of a subsequent frame are illustrated. It should be understood that each subsequent frame can be structured similarly to frame 458. Further, the transmission mode of the radar subsystem may be fixed. That is, regardless of whether a user is present or not, the time of day, or other factors, chirps may be transmitted according to chirp timing diagram 400. Therefore, in some embodiments, the radar subsystem always operates in a single transmission mode, regardless of the state of the environment or the activity attempting to be monitored. A continuous train of frames similar to frame 458 may be transmitted while device 101 is powered on.

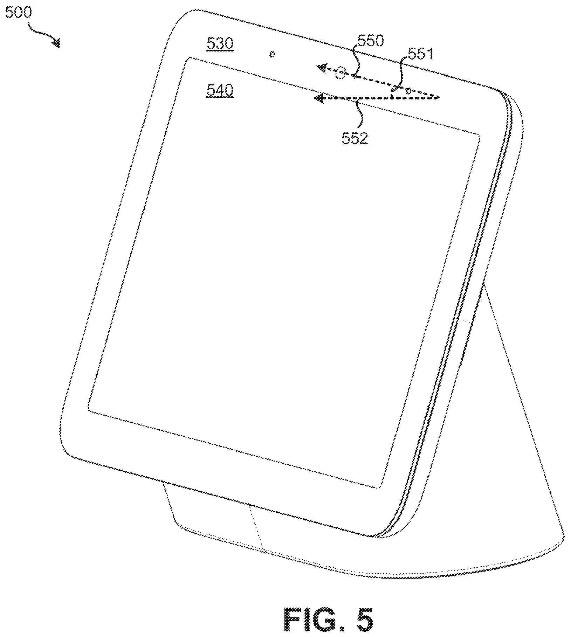

**FIG.** 5 illustrates an embodiment of a contactless BP measurement device 500 ("device 500"). Device 500 can be a smart home hub device that can receive voice-based commands and provide an interface for a user to interact with various other smart home devices, view pictures, interact with media, and perform various other tasks. Device 500 may have a front surface that includes a front transparent screen 540 such that a display is visible. Such a display may be a touchscreen. Surrounding front transparent screen 540 may be an optically opaque region, referred to as bezel 530, through which radar subsystem 205 may have a field-of-view of the environment in front of device 500.

For purposes of the immediate following description, the terms vertical and horizontal describe directions relative to the bedroom in general, with vertical referring to a direction perpendicular to the floor and horizontal referring to a direction parallel to the floor. Since the radar subsystem, which may be an Infineon^{®} BGT60 radar chip, is roughly planar and is installed generally parallel to bezel 530 for spatial compactness of the device as a whole, and since the antennas within the radar chip lie in the plane of the chip, then, without beam targeting, a receive beam of radar subsystem 120 may be pointed in direction 550 that is generally normal to bezel 530. Due to a departure tilt of bezel 530 away from a purely vertical direction, which is provided in some embodiments to be about 25 degrees in order to facilitate easy user interaction with a touchscreen functionality of the transparent screen 540, direction 550 may point upwards from horizontal by departure angle 551. Assuming device 500 will typically be installed on a bedside platform (e.g., nightstand) that is roughly the same height as the top of a mattress on which a user will lie down, it may be beneficial for the receive beam of radar subsystem 120 to be targeted in horizontal direction 552 or an approximately horizontal (e.g., between -5° and 5° from horizontal) direction. Therefore, vertical beam targeting can be used to compensate for departure angle 551 of the portion of device 500 in which radar subsystem 120 is present.

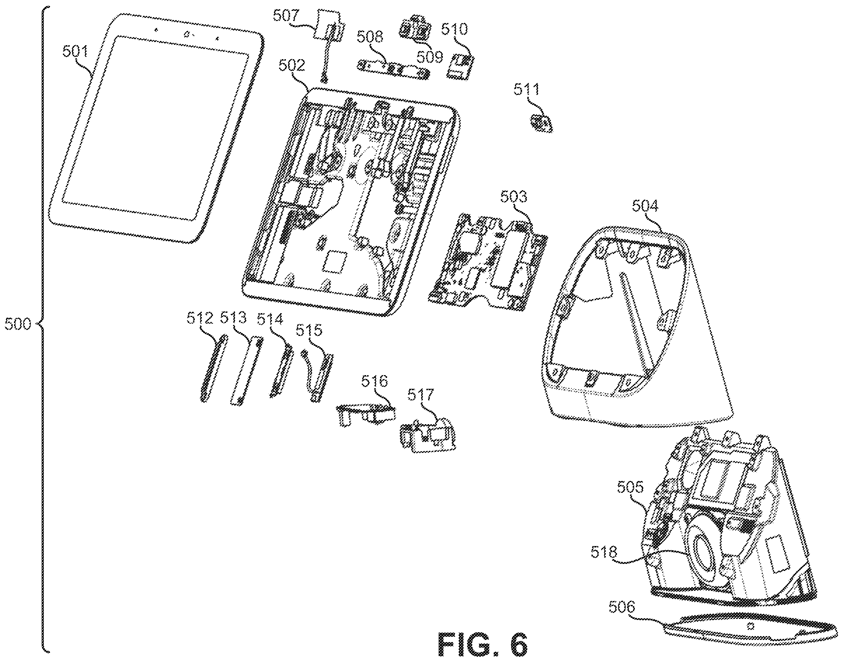

**FIG. 6** illustrates an exploded view of an embodiment of contactless BP measurement device 500. Device 500 can include: display assembly 501; display housing 502; main circuit board 503; neck assembly 504; speaker assembly 505; base plate 506; mesh network communication interface 507; top daughterboard 508; button assembly 509; radar assembly 510; microphone assembly 511; rocker switch bracket 512; rocker switch board 513; rocker switch button 514; Wi-Fi assembly 515; power board 516; and power bracket assembly 517. Device 500 can represent an embodiment of how device 101 may be implemented.

Display assembly 501, display housing 502, neck assembly 504, and base plate 506 may collectively form a housing that houses all of the remaining components of device 500. Display assembly 501 may include an electronic display, which can be a touchscreen, that presents information to a user. Display assembly 501 may, therefore, include a display screen, which can include a metallic plate of the display that can serve as a grounding plane. Display assembly 501 may include transparent portions away from the metallic plate that allow various sensors a field of view in the general direction in which display assembly 501 is facing. Display assembly 501 may include an outer surface made of glass or transparent plastic that serves as part of the housing of device 500.

Display housing 502 may be a plastic or other rigid or semi-rigid material that serves as a housing for display assembly 501. Various components, such as main circuit board 503; mesh network communication interface 507; top daughterboard 508; button assembly 509; radar assembly 510; and microphone assembly 511 may be mounted on display housing 502. Mesh network communication interface 507; top daughterboard 508; radar assembly 510; and microphone assembly 511 may be connected to main circuit board 503, using flat wire assemblies. Display housing may be attached with display assembly 501, using an adhesive.

Mesh network communication interface 507 may include one or more antennas and may enable communication with a mesh network, such as a Thread-based mesh network. Wi-Fi assembly 515 may be located a distance from mesh network communication interface 507 to decrease the possibility of interference. Wi-Fi assembly 515 may enable communication with a Wi-Fi based network.

Radar assembly 510, which can include radar subsystem 120 or radar subsystem 205, may be positioned such that its RF emitter and RF receiver are away from the metallic plate of display assembly 501 and are located a significant distance from mesh network communication interface 507 and Wi-Fi assembly 515. These three components may be arranged in approximately a triangle to increase the distance between the components and decrease interference. For instance, in device 500, a distance of at least 74 mm between Wi-Fi assembly 515 and radar assembly 510 may be maintained. A distance of at least 98 mm between mesh network communication interface 507 and radar assembly 510 may be maintained. Additionally, distance between radar assembly 510 and speaker 518 may be desired to minimize the effect of vibrations on radar assembly 510 that may be generated by speaker 518. For instance, for device 500, a distance of at least 79 mm between radar assembly 510 and speaker 518 may be maintained. Additionally, distance between the microphones and radar assembly 510 may be desired to minimize any possible interference from the microphones on received radar signals. Top daughterboard 508 may include multiple microphones. For instance, at least 12 mm may be maintained between a closest microphone of top daughterboard 508 and radar assembly 510.

Other components may also be present. A third microphone assembly may be present, microphone assembly 511, which may be rear-facing. Microphone assembly 511 may function in concert with the microphones of top daughterboard 508 to isolate spoken commands from background noise. Power board 516 may convert power received from an AC power source to DC to power the components of device 500. Power board 516 may be mounted within device 500 using power bracket assembly 517. Rocker switch bracket 512, rocker switch board 513, and rocker switch button 514 may be collectively used to receive user input, such as up/down input. Such input may be used, for example, to adjust a volume of sound output through speaker 518. As another user input, button assembly 509 may include a toggle button that a user can actuate. Such a user input may be used to activate and deactivate all microphones, such as for when the user desires privacy and/or does not want device 500 to respond to voice commands.

In order to accurately measure PTT, the BP measurement device needs to accurately measure when the PPW is present at or near the aortic valve of the user and when the PPW arrives at an extremity of the user. FIG. 7 illustrates an embodiment 700 of a user having the user's BP measured using a radar-based BP measurement system. Contactless BP measurement device 500, which can represent an embodiment of BP measurement device 101, can be stationary on a surface, such as table 730. User 720 may be instructed to place one or both hands in front of their body, possibly having the user's arms supported by the table to help the user keep the user's hands relatively motionless. In this position, the user's hands 722 are at distance 701 from contactless BP measurement device 500, which is different from distance 702 of the user's aortic valve 724, thus allowing the radar sensor of contactless BP device 500 to separately detect the PPW at aortic valve 724 and at hand 722.

Additionally or alternatively, a user may have the user's BP measured while lying down. **FIG. 8** illustrates an embodiment 800 of user 720 having the user's BP measured using a radar-based BP measurement system. Contactless BP measurement device 500, which can represent an embodiment of BP measurement device 101, can be stationary on a surface, such as nightstand 830. User 720 may be instructed to lie down on a surface (e.g., bed 832) such that the user's feet 810 are extended toward BP measurement device 500. In this position, the user's feet 810 are at distance 801 from contactless BP measurement device 500, which is different from distance 802 from BP measurement device 500 to the user's aortic valve 820, thus allowing the radar sensor of contactless BP device 500 to separately detect the PPW at aortic valve 820 and at feet 810. Other positions are also possible. Since blanket 835 remains motionless or near motionless during the BP measurement and allows RF to pass through without much loss, it may have a negligible effect on the BP measurement.

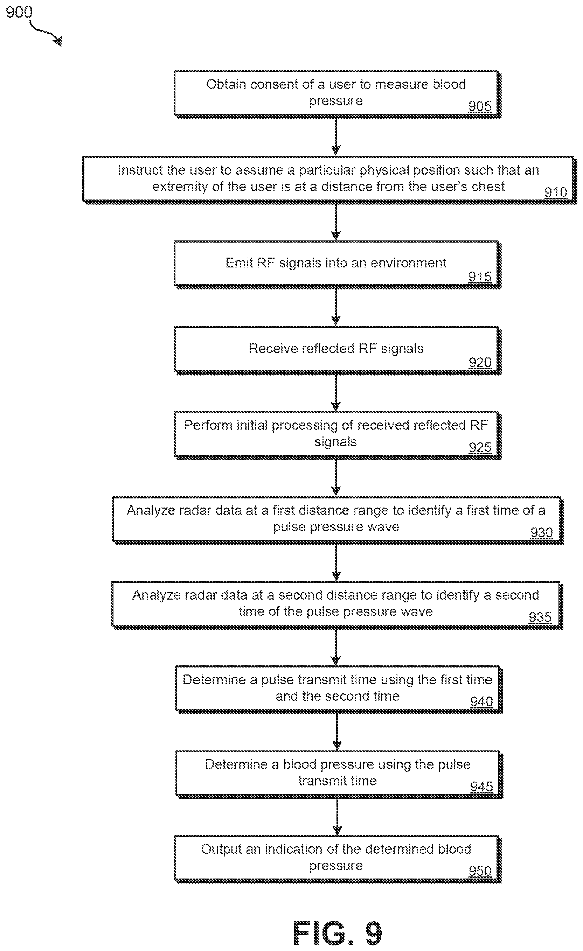

Various methods can be performed using the systems, devices, and arrangements of FIGS. 1-8. FIG. 9 illustrates an embodiment of a method 900 for measuring BP using a contactless BP measurement device, such as detailed in relation to FIG. 1. The BP measurement device used to perform method 900 can include system 200. At block 905, consent may be obtained to measure the blood pressure of the user. Block 805 may include the user providing a vocal command requesting the user's blood pressure be measured or inputting such a command to the BP measurement device, such as via a touchscreen. The consent may inform the user that their BP will be measured and, possibly, the resulting measurement stored in association with a user account of the user. For a first BP measurement, a user may be required to review and agree with a detailed end-user agreement. Method 900 may only proceed if consent is provided by the user. Further, since method 900 requires the user to remain in a particular physical position, consent can be inferred from the user assuming the physical position and remaining still in that position to allow the BP measurement to be made.

At block 910, instructions may be output using synthesized speech, via a message presented on a display of the BP measurement device, or both that instructs the user to be in a particular physical position. Such a position can involve an extremity of the user being a set distance from the radar sensor of the BP measurement device. For example, the user may be instructed to place the user's hands or feet approximately 1 foot (0.3 m) from the radar sensor. Examples of possible physical positions are presented in relation to FIGS. 7 and 8. A key aspect is that the user's chest (and, more specifically, the user's aortic valve) is at a different distance from the radar sensor than the extremities being used as part of the BP measurement. FIGS. 7 and 8 illustrate examples where the extremities are hands and feet, respectively; however, in other embodiments, another extremity may be possible, such as the user's face. (PTT from the aortic valve to any extremity of the user is similar enough to use for measurement of BP.) Following block 910, the user may be still and in a physical position that allows for a PPW to be detected at or near the user's aortic valve and at the extremity of the user using radar.

At block 915, RF signals are emitted into an environment by the BP measurement device, such as by radar subsystem 205. The RF signals may be FMCW radar, as detailed in relation to FIG. 4. Reflections of the emitted RF signals are received at block 920. At block 925, initial processing of the received RF signals are performed to obtain distance-binned frequency measurements, as detailed in relation to radar processing module 210. Further detail regarding the processing of the received RF signals can be found detailed in relation to FIG. 2.

At block 930, the radar data is analyzed at a first distance range by the BP measurement device. The first distance range corresponds to a distance bin at which the user's aortic valve is present. Since the aortic value moves a significant distance with each heartbeat compared to blood vessels, the distance bin at which a highest magnitude frequency component is present that corresponds to a resting heart rate (e.g., between 40 and 150 BPM) may be selected. A first time at which a PPW originates may be stored. In some embodiments, block 930 involves applying a trained machine learning model, such as a trained neural network, to the radar data to determine the first distance range and/or the time at which the PPW is detected within the first distance range.

At block 935, the radar data is analyzed at a second distance range by the BP measurement device. The second distance range corresponds to a distance bin at which the user's extremity is present. In some embodiments, the user is requested to hold their extremity a fixed distance from the radar sensor; therefore, the distance bin may be predetermined. In other embodiments, since the extremity is extended toward the radar sensor, the closest distance at which a frequency component is detected that corresponds to the resting heart rate may be selected. A second time at which the PPW is detected may be stored. In some embodiments, block 935 involves applying a trained machine learning model, such as a trained neural network, to the radar data to determine the second distance range and/or the time at which the PPW is detected within the second distance range.

At block 930 and 935, in addition to analyzing the radar data to measure the time of a PPW originating and arriving at an extremity, the DPWA and/or HR of the user may be measured at the user's aortic valve, extremity, or both.

Following block 930 and block 935, an amount of time that elapses from when a PPW originated at the aortic value to when the PPW reached the extremity of the user can be determined by the BP measurement device at block 940. This difference in time is the PPT. Blocks 915 through 940 may be repeated for a period of time and averaged or otherwise combined together to obtain a more accurate PPT.

The PPT, possibly along with the DPWA and/or HR, can be used to determine the BP (systolic and diastolic) of the user at block 945. In some embodiments, a look-up table or algorithm is used that takes the PPT, DPWA, and/or HR as inputs and outputs the BP. In other embodiments, a trained machine learning model can take the PPT, DPWA, and/or HR as inputs and outputs the BP values. At block 950, an indication of the determined blood pressure is output. The indication of the determined BP can be output via synthesized speech, via a display of the BP measurement device, and/or output to a cloud-based server system (e.g., for storage or access by the user from another computerized device).

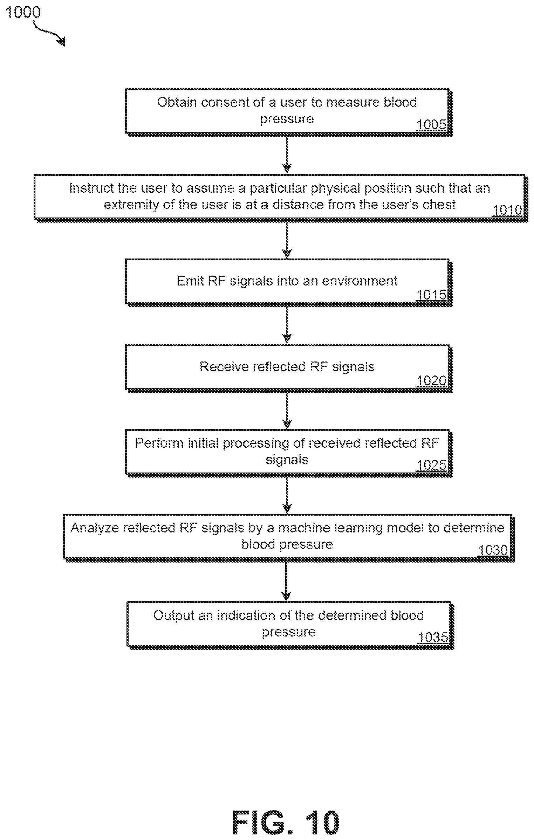

**FIG. 10** illustrates an embodiment of method 1000 for measuring BP using a contactless BP measurement device, such as detailed in relation to FIG. 1. The BP measurement device used to perform method 900 can include system 300 of FIG. 3. At block 1005, consent may be obtained to measure the blood pressure of the user. Block 1005 may include the user providing a vocal command requesting the user's blood pressure be measured or inputting such a command to the BP measurement device, such as via a touchscreen. The consent may inform the user that their BP will be measured and, possibly, the resulting measurement stored in association with a user account of the user. For a first BP measurement, a user may be required to review and agree with a detailed end-user agreement. Method 1000 may only proceed if consent is provided by the user. Further, since method 1000 requires the user to remain in a particular physical position, consent can be inferred from the user assuming the physical position and remaining still in that position to allow the BP measurement to be made.

At block 1010, instructions may be output using synthesized speech, via a message presented on a display of the BP measurement device, or both that instructs the user to be in a particular physical position. Such a position can involve an extremity of the user being a set distance from the radar sensor of the BP measurement device. For example, the user may be instructed to place the user's hands or feet approximately 1 foot (0.3 m) from the radar sensor. Examples of possible physical positions are presented in relation to FIGS. 7 and 8. A key aspect is that the user's chest (and, more specifically, the user's aortic valve) is at a different distance from the radar sensor than the extremities being used as part of the BP measurement. FIGS. 7 and 8 illustrate examples where the extremities are hands and feet, respectively; however, in other embodiments, another extremity may be possible, such as the user's face. (PTT from the aortic valve to any extremity of the user is similar enough to use for measurement of BP.) Following block 910, the user may be still and in a physical position that allows for a PPW to be detected at or near the user's aortic valve and at the extremity of the user using radar.

At block 1015, RF signals may be emitted into an environment by the BP measurement device, such as by radar subsystem 205. The RF signals may be FMCW radar, as detailed in relation to FIG. 4. Reflections of the emitted RF signals may be received at block 1020. At block 1025, initial processing of the received RF signals are performed to obtain distance-binned frequency measurements, as detailed in relation to radar processing module 210. Further detail regarding the processing of the received RF signals can be found detailed in relation to FIG. 2.

At block 1030, the radar data may be analyzed by a machine learning model. The machine learning model may be trained to use the radar data from the radar subsystem as in input and output a blood pressure (systolic and diastolic) of the user. Therefore, the trained machine learning model directly analyzes the radar data to determine BP. The machine learning model may be a trained neural network. In some embodiments, an additional layer may be added or altered at the BM measurement device to factor in a calibration profile detailed in relation to FIG. 11.

At block 1035, an indication of the determined blood pressure is output. The indication of the determined BP can be output via synthesized speech, via a display of the BP measurement device, and/or output to a cloud-based server system (e.g., for storage or access by the user from another computerized device).

To accurately determine BP using PTT, HR, and/or DPWA, a calibration process may be completed for an individual user. The calibration process can account for the specific physical position the user will be in when the BP measurements are made via radar and/or characteristics particular to the user. **FIG. 11** illustrates an embodiment of a method 1100 for calibrating a radar-based BP measurement system. In method 1100, prior to using methods 900 or 1000 to determine an accurate BP, the user may be instructed to use a separate blood pressure measurement device (that is expected to be accurate) to determine the user's BP at block 1105. The separate blood pressure device may use a mercury-gravity manometer, an aneroid gauge, or an electronic device. Such devices can use a cuff placed around an arm to measure blood pressure or a sensor placed on a finger. In other embodiments, a combination of an electrocardiogram (ECG) and finger photoplethysmography (PPG) may be used to determine PPT. The determined BP measurements may be input to the BP measurement device or to the cloud-based server system in communication with the BP measurement device via a computerized device (e.g., smartphone) at block 1110.

Prior to block 1105 or after block 1105 or block 1110, a user may then have their BP measured according to method 900 or method 1000. Ideally, the two BP measurements are made within a short period of time of each other. For example, the user may perform one BP measurement and immediately perform the other, possibly while remaining seated or lying down. Whichever physical position the user uses (e.g., from FIG. 7 or FIG. 8), the user may be instructed to continue to use the same position for future BP measurements made by the BP measurement device.

At block 1115, the BP measured by the BP measurement device can be compared with the received BP measurement from block 1110. In some embodiments, the user is required or requested to perform multiple rounds of BP measurements using the BP measurement device and the separate BP measurement device to allow for a greater number of comparisons. The one or more comparisons performed at block 1115 are used to create a calibration profile at block 1120. In some embodiments, blood pressure measurements made via the separate blood pressure device and the corresponding BP measurements using the BP measurement device of FIG. 1 are used to create a training data set that can be used to optimize the machine learning models used in systems 200 and 300 or methods 900 and 1000.

In some embodiments, the calibration profile can be simple offset values, an offset algorithm, or offset look-up that gets created. In other embodiments, a calibration model can be created, such as using one or few-shot learning, maximum likelihood estimation, or least-squares estimation.

The created calibration profile or calibration training data set can be used to adjust BP measurements made using the BP measurement system, such as in method 900 or method 1000. In method 900, the calibration profile or calibration training data set may be used as part of block 945 to adjust the BP measurement for the user. Referring to system 200, this calibration may be performed by BP determination engine 224. In method 1000, the calibration training data set may be used to create or adjust a layer of the machine learning model used at block 1030. Referring to system 300, analysis engine 311 may be modified based on the calibration profile.

It should be noted that the methods, systems, and devices discussed above are intended merely to be examples. It must be stressed that various embodiments may omit, substitute, or add various procedures or components as appropriate. For instance, it should be appreciated that, in alternative embodiments, the methods may be performed in an order different from that described, and that various steps may be added, omitted, or combined. Also, features described with respect to certain embodiments may be combined in various other embodiments. Different aspects and elements of the embodiments may be combined in a similar manner. Also, it should be emphasized that technology evolves and, thus, many of the elements are examples and should not be interpreted to limit the scope of the invention.

Specific details are given in the description to provide a thorough understanding of the embodiments. However, it will be understood by one of ordinary skill in the art that the embodiments may be practiced without these specific details. For example, well-known processes, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing embodiments of the invention.

Also, it is noted that the embodiments may be described as a process which is depicted as a flow diagram or block diagram. Although each may describe the operations as a sequential process, many of the operations can be performed in parallel or concurrently. In addition, the order of the operations may be rearranged. A process may have additional steps not included in the figure.

For example, the above elements may merely be a component of a larger system, wherein other rules may take precedence over or otherwise modify the application of the invention. Also, a number of steps may be undertaken before, during, or after the above elements are considered.

## Claims

1. A method (900; 1000) for measuring blood pressure, comprising:
emitting, by a radar sensor (120;205), radio frequency (RF) signals;
receiving, by the radar sensor, RF reflection signals based on the emitted RF signals being reflected, wherein the radar sensor is included in a contactless blood pressure measurement device (101;500);
processing, by a processing system (110), the received RF signals to obtain distance-binned frequency measurements;
analyzing, by the processing system, the processed received RF reflection signals at a first distance range (702;802) corresponding to a first distance bin to identify a first time of a pulse pressure wave at an aortic valve (724; 820) of a user (720);
analyzing, by the processing system, the processed received RF reflection signals at a second distance range (701;801) different from the first distance range and corresponding to a second distance bin to identify a second time of the pulse pressure wave at an extremity (710;810) of the user;
determining, by the processing system, a pulse transmit time (PTT) of the pulse pressure wave from the aortic valve of the user to the extremity of the user using the first time and the second time;
determining, by the processing system, a blood pressure of the user based on the determined PTT; and
outputting, by the processing system, an indication of the determined blood pressure.

2. The method for measuring blood pressure of claim 1, wherein the extremity of the user is one or more hands of the user or wherein the extremity of the user is one or more feet of the user.

3. The method for measuring blood pressure of claim 1, wherein analyzing the RF reflection signals at the first distance range and at the second distance range is performed by one or more neural networks.

4. The method for measuring blood pressure of claim 1, further comprising determining a heart rate based on analyzing the RF reflection signals, wherein determining the blood pressure of the user is further based on the heart rate
or
further comprising determining a derived pulse waveform amplitude (DPWA) at the extremity of the user based on analyzing the RF reflection signals, wherein determining the blood pressure of the user is further based on the DPWA.

5. The method for measuring blood pressure of claim 1, further comprising:
receiving, by the processing system, an external blood pressure measurement made using a blood pressure device separate from a device (101;500) comprising the radar sensor;
comparing, by the processing system, the external blood pressure measurement and the determined blood pressure measurement; and
creating, by the processing system, a calibration profile for use in modifying a future determined blood pressure measurement,
wherein, optionally, a machine learning model is modified based on the calibration profile specific to the user.

6. The method for measuring blood pressure of claim 1, wherein the radar sensor and the processing system are integrated as part of a home assistant hub device that further comprises a display screen and speaker, wherein the indication of the determined blood pressure is output using the display screen, the speaker, or both.

7. The method for measuring blood pressure of claim 1, wherein the contactless blood pressure measurement device is configured to be stationary on a surface (730;830).

8. A blood pressure measurement system (100;200;300), comprising:
a contactless blood pressure measurement device (101;500);
a radar subsystem (120; 205) included in the contactless blood pressure measurement device, comprising:
a radio frequency (RF) emitter (206) that emits RF signals;
an RF receiver (207) that receives RF reflection signals based on the emitted RF signals being reflected; and
a processing system (110), comprising one or more processors, in communication with the radar subsystem, wherein the processing system is configured to:
process the received RF signals to obtain distance-binned
frequency measurements,
analyze the processed received RF reflection signals at a first distance range (702; 802) corresponding to a first distance bin to identify a first time of a pulse pressure wave at an aortic valve (724;820) of a user (720);
analyze the processed received RF reflection signals at a second distance range (701; 801) different from the first distance range and corresponding to a second distance bin to identify a second time of the pulse pressure wave at an extremity (722; 810) of the user;
determine a pulse transmit time (PTT) of the pulse pressure wave from the aortic valve of the user to the extremity of the user using the first time and the second time;
determine a blood pressure of the user based on the determined PTT; and
output an indication of the determined blood pressure.

9. The blood pressure measurement system of claim 8, further comprising a housing, wherein the housing houses the radar subsystem and the processing system
wherein, optionally, the housing further houses: a microphone, a speaker (518), and an electronic display, wherein the indication of the determined blood pressure is output via the electronic display.

10. The blood pressure measurement system of claim 8, wherein the extremity of the user is one or more hands of the user or
wherein the extremity of the user is one or more feet of the user.

11. The blood pressure measurement system of claim 8, wherein analyzing the RF reflection signals at the first distance range and at the second distance range is performed by one or more neural networks.

12. The blood pressure measurement system of claim 8, wherein the processing system is further configured to determine a heart rate based on analyzing the RF reflection signals, wherein determining the blood pressure of the user is further based on the heart rate.

13. The blood pressure measurement system of claim 8, wherein the processing system is further configured to determine a derived pulse waveform amplitude (DPWA) at the extremity of the user based on analyzing the RF reflection signals, wherein determining the blood pressure of the user is further based on the DPWA.

14. The blood pressure measurement system of claim 8, wherein the processing system is further configured to:
receive an external blood pressure measurement made using a blood pressure device separate from a device comprising the radar subsystem;
compare the external blood pressure measurement and the determined blood pressure measurement; and
create a calibration profile for use in modifying a future determined blood pressure measurement,
wherein, optionally, a machine learning model is modified based on the calibration profile specific to the user.

15. A non-transitory processor-readable medium, comprising processor-readable instructions configured to cause one or more processors to:
process radio frequency (RF) reflection signals to obtain distance-binned frequency measurements, wherein the RF reflection signals are received from a radar sensor (120;205) included in a contactless blood pressure measurement device (101;500);
analyze the radio frequency (RF) reflection signals at a first distance (702;801) range corresponding to a first distance bin to identify a first time of a pulse pressure wave at an aortic valve (724;810) of a user (720);
analyze the processed RF reflection signals at a second distance range (701;801) different from the first distance range and corresponding to a second distance bin to identify a second time of the pulse pressure wave at an extremity (710;810) of the user;
determine a pulse transmit time (PTT) of the pulse pressure wave from the aortic valve of the user to the extremity of the user using the first time and the second time;
determine a blood pressure of the user based on the determined PTT; and output an indication of the determined blood pressure.

## Patentansprüche

1. Verfahren (900; 1000) zur Messung des Blutdrucks, umfassend:
Emittieren, durch einen Radarsensor (120; 205), von Hochfrequenz-Signalen (HF-Signalen);
Empfangen, durch den Radarsensor, von HF-Reflexionssignalen basierend darauf, dass die emittierten HF-Signale reflektiert werden, wobei der Radarsensor in einer kontaktlosen Blutdruckmessvorrichtung (101; 500) enthalten ist;
Verarbeiten, durch ein Verarbeitungssystem (110), der empfangenen HF-Signale, um in Entfernungs-Bins eingeteilte Frequenzmessungen zu erhalten;
Analysieren, durch das Verarbeitungssystem, der verarbeiteten empfangenen HF-Reflexionssignale in einem ersten Entfernungsbereich (702; 802), der einem ersten Entfernungs-Bin entspricht, um eine erste Zeit einer Pulsdruckwelle an einer Aortenklappe (724; 820) eines Benutzers (720) zu identifizieren;
Analysieren, durch das Verarbeitungssystem, der verarbeiteten empfangenen HF-Reflexionssignale in einem zweiten Entfernungsbereich (701; 801), der sich von dem ersten Entfernungsbereich unterscheidet und einem zweiten Entfernungs-Bin entspricht, um eine zweite Zeit der Pulsdruckwelle an einer Extremität (710; 810) des Benutzers zu identifizieren;
Bestimmen, durch das Verarbeitungssystem, einer Pulsübertragungszeit (pulse transmit time, PTT) der Pulsdruckwelle von der Aortenklappe des Benutzers zu der Extremität des Benutzers unter Verwendung der ersten Zeit und der zweiten Zeit;
Bestimmen, durch das Verarbeitungssystem, eines Blutdrucks des Benutzers basierend auf der bestimmten PTT; und
Ausgeben, durch das Verarbeitungssystem, einer Angabe des bestimmten Blutdrucks.

2. Verfahren zur Messung des Blutdrucks nach Anspruch 1, wobei die Extremität des Benutzers eine oder mehrere Hände des Benutzers ist oder wobei die Extremität des Benutzers ein oder mehrere Füße des Benutzers ist.

3. Verfahren zur Messung des Blutdrucks nach Anspruch 1, wobei das Analysieren der HF-Reflexionssignale in dem ersten Entfernungsbereich und in dem zweiten Entfernungsbereich durch ein oder mehrere neuronale Netzwerke durchgeführt wird.

4. Verfahren zur Messung des Blutdrucks nach Anspruch 1, ferner umfassend das Bestimmen einer Herzfrequenz basierend auf dem Analysieren der HF-Reflexionssignale, wobei das Bestimmen des Blutdrucks des Benutzers ferner auf der Herzfrequenz basiert oder
ferner umfassend das Bestimmen einer abgeleiteten Pulswellenformamplitude (derived pulse waveform amplitude, DPWA) an der Extremität des Benutzers basierend auf dem Analysieren der HF-Reflexionssignale, wobei das Bestimmen des Blutdrucks des Benutzers ferner auf der DPWA basiert.

5. Verfahren zur Messung des Blutdrucks nach Anspruch 1, ferner umfassend:
Empfangen, durch das Verarbeitungssystem, einer externen Blutdruckmessung, die unter Verwendung einer Blutdruckvorrichtung durchgeführt wurde, die getrennt von einer Vorrichtung (101; 500) ist, die den Radarsensor umfasst;
Vergleichen, durch das Verarbeitungssystem, der externen Blutdruckmessung und der bestimmten Blutdruckmessung; und
Erstellen, durch das Verarbeitungssystem, eines Kalibrierungsprofils zur Verwendung beim Modifizieren einer zukünftig bestimmten Blutdruckmessung,
wobei, optional, ein maschinelles Lernmodell basierend auf dem für den Benutzer spezifischen Kalibrierungsprofil modifiziert wird.

6. Verfahren zur Messung des Blutdrucks nach Anspruch 1, wobei der Radarsensor und das Verarbeitungssystem als Teil einer Heimassistenten-Hub-Vorrichtung integriert sind, die ferner einen Anzeigebildschirm und einen Lautsprecher umfasst, wobei die Angabe des bestimmten Blutdrucks unter Verwendung des Anzeigebildschirms, des Lautsprechers oder beider ausgegeben wird.

7. Verfahren zur Messung des Blutdrucks nach Anspruch 1, wobei die kontaktlose Blutdruckmessvorrichtung dafür konfiguriert ist, stationär auf einer Oberfläche (730; 830) zu sein.

8. Blutdruckmesssystem (100; 200; 300), umfassend:
eine kontaktlose Blutdruckmessvorrichtung (101; 500);
ein Radar-Subsystem (120; 205), das in der kontaktlosen Blutdruckmessvorrichtung enthalten ist, umfassend:
einen Hochfrequenz-Emitter (HF-Emitter) (206), der HF-Signale emittiert;
einen HF-Empfänger (207), der HF-Reflexionssignale basierend darauf empfängt, dass die emittierten HF-Signale reflektiert werden; und
ein Verarbeitungssystem (110), das einen oder mehrere Prozessoren umfasst und in Kommunikation mit dem Radar-Subsystem steht, wobei das Verarbeitungssystem für Folgendes konfiguriert ist:
Verarbeiten der empfangenen HF-Signale, um in Entfernungs-Bins eingeteilte Frequenzmessungen zu erhalten,
Analysieren der verarbeiteten empfangenen HF-Reflexionssignale in einem ersten Entfernungsbereich (702; 802), der einem ersten Entfernungs-Bin entspricht, um eine erste Zeit einer Pulsdruckwelle an einer Aortenklappe (724; 820) eines Benutzers (720) zu identifizieren;
Analysieren der verarbeiteten empfangenen HF-Reflexionssignale in einem zweiten Entfernungsbereich (701; 801), der sich von dem ersten Entfernungsbereich unterscheidet und einem zweiten Entfernungs-Bin entspricht, um eine zweite Zeit der Pulsdruckwelle an einer Extremität (722; 810) des Benutzers zu identifizieren;
Bestimmen einer Pulsübertragungszeit (PTT) der Pulsdruckwelle von der Aortenklappe des Benutzers zu der Extremität des Benutzers unter Verwendung der ersten Zeit und der zweiten Zeit;
Bestimmen eines Blutdrucks des Benutzers basierend auf der bestimmten PTT; und
Ausgeben einer Angabe des bestimmten Blutdrucks.

9. Blutdruckmesssystem nach Anspruch 8, ferner umfassend ein Gehäuse, wobei das Gehäuse das Radar-Subsystem und das Verarbeitungssystem aufnimmt
wobei, optional, das Gehäuse ferner Folgendes aufnimmt: ein Mikrofon, einen Lautsprecher (518) und eine elektronische Anzeige, wobei die Angabe des bestimmten Blutdrucks über die elektronische Anzeige ausgegeben wird.

10. Blutdruckmesssystem nach Anspruch 8, wobei die Extremität des Benutzers eine oder mehrere Hände des Benutzers ist oder wobei die Extremität des Benutzers ein oder mehrere Füße des Benutzers ist.

11. Blutdruckmesssystem nach Anspruch 8, wobei das Analysieren der HF-Reflexionssignale in dem ersten Entfernungsbereich und in dem zweiten Entfernungsbereich durch ein oder mehrere neuronale Netzwerke durchgeführt wird.

12. Blutdruckmesssystem nach Anspruch 8, wobei das Verarbeitungssystem ferner dafür konfiguriert ist, eine Herzfrequenz basierend auf dem Analysieren der HF-Reflexionssignale zu bestimmen, wobei das Bestimmen des Blutdrucks des Benutzers ferner auf der Herzfrequenz basiert.

13. Blutdruckmesssystem nach Anspruch 8, wobei das Verarbeitungssystem ferner dafür konfiguriert ist, eine abgeleitete Pulswellenformamplitude (DPWA) an der Extremität des Benutzers basierend auf dem Analysieren der HF-Reflexionssignale zu bestimmen, wobei das Bestimmen des Blutdrucks des Benutzers ferner auf der DPWA basiert.

14. Blutdruckmesssystem nach Anspruch 8, wobei das Verarbeitungssystem ferner für Folgendes konfiguriert ist:
Empfangen einer externen Blutdruckmessung, die unter Verwendung einer Blutdruckvorrichtung durchgeführt wurde, die getrennt von einer Vorrichtung ist, die das Radar-Subsystem umfasst;
Vergleichen der externen Blutdruckmessung und der bestimmten Blutdruckmessung; und
Erstellen eines Kalibrierungsprofils zur Verwendung beim Modifizieren einer zukünftig bestimmten Blutdruckmessung, wobei, optional, ein maschinelles Lernmodell basierend auf dem für den Benutzer spezifischen Kalibrierungsprofil modifiziert wird.

15. Beständiges, prozessorlesbares Medium, umfassend prozessorlesbare Anweisungen, die dafür konfiguriert sind, einen oder mehrere Prozessoren zu Folgendem zu veranlassen:
Verarbeiten von Hochfrequenz-Reflexionssignalen (HF-Reflexionssignalen), um in Entfernungs-Bins eingeteilte Frequenzmessungen zu erhalten, wobei die HF-Reflexionssignale von einem Radarsensor (120; 205) empfangen werden, der in einer kontaktlosen Blutdruckmessvorrichtung (101; 500) enthalten ist;
Analysieren der Hochfrequenz-Reflexionssignale (HF-Reflexionssignale) in einem ersten Entfernungsbereich (702; 801), der einem ersten Entfernungs-Bin entspricht, um eine erste Zeit einer Pulsdruckwelle an einer Aortenklappe (724; 810) eines Benutzers (720) zu identifizieren;
Analysieren der verarbeiteten HF-Reflexionssignale in einem zweiten Entfernungsbereich (701; 801), der sich von dem ersten Entfernungsbereich unterscheidet und einem zweiten Entfernungs-Bin entspricht, um eine zweite Zeit der Pulsdruckwelle an einer Extremität (710; 810) des Benutzers zu identifizieren; Bestimmen einer Pulsübertragungszeit (PTT) der Pulsdruckwelle von der Aortenklappe des Benutzers zu der Extremität des Benutzers unter Verwendung der ersten Zeit und der zweiten Zeit; Bestimmen eines Blutdrucks des Benutzers basierend auf der bestimmten PTT; und Ausgeben einer Angabe des bestimmten Blutdrucks.

## Revendications

1. Procédé (900 ; 1000) de mesure de la pression artérielle, comprenant :
l'émission, par un capteur radar (120 ; 205), de signaux radiofréquence (RF) ;
la réception, par le capteur radar, de signaux de réflexion RF sur la base des signaux RF émis étant réfléchis, dans lequel le capteur radar est inclus dans un dispositif de mesure de la pression artérielle sans contact (101 ; 500) ;
le traitement, par un système de traitement (110), des signaux RF reçus pour obtenir des mesures de fréquence regroupées par distance ;
l'analyse, par le système de traitement, des signaux de réflexion RF reçus traités à une première plage de distance (702 ; 802) correspondant à une première classe de distance pour identifier un premier temps d'une onde de pression d'impulsion au niveau d'une valve aortique (724 ; 820) d'un utilisateur (720) ;
l'analyse, par le système de traitement, des signaux de réflexion RF reçus traités à une seconde plage de distance (701 ; 801) différente de la première plage de distance et correspondant à une seconde classe de distance pour identifier un second temps de l'onde de pression d'impulsion au niveau d'une extrémité (710 ; 810) de l'utilisateur ;
la détermination, par le système de traitement, d'un temps de transmission d'impulsion (pulse transmit time, PTT) de l'onde de pression d'impulsion de la valve aortique de l'utilisateur jusqu'à l'extrémité de l'utilisateur à l'aide du premier temps et du second temps ;
la détermination, par le système de traitement, de la pression artérielle de l'utilisateur sur la base du PTT déterminé ; et la fourniture, par le système de traitement, d'une indication de la pression artérielle déterminée.

2. Procédé de mesure de la pression artérielle selon la revendication 1, dans lequel l'extrémité de l'utilisateur est une ou plusieurs mains de l'utilisateur ou dans lequel l'extrémité de l'utilisateur est un ou plusieurs pieds de l'utilisateur.

3. Procédé de mesure de la pression artérielle selon la revendication 1, dans lequel l'analyse des signaux de réflexion RF à la première plage de distance et à la seconde plage de distance est réalisée par un ou plusieurs réseaux neuronaux.

4. Procédé de mesure de la pression artérielle selon la revendication 1, comprenant en outre la détermination d'une fréquence cardiaque sur la base d'une analyse des signaux de réflexion RF, dans lequel la détermination de la pression artérielle de l'utilisateur est en outre sur la base de la fréquence cardiaque
ou
comprenant en outre la détermination d'une amplitude de forme d'onde de pouls dérivée (derived pulse waveform amplitude, DPWA) au niveau de l'extrémité de l'utilisateur sur la base de l'analyse des signaux de réflexion RF, dans lequel la détermination de la pression artérielle de l'utilisateur est en outre sur la base de la DPWA.

5. Procédé de mesure de la pression artérielle selon la revendication 1, comprenant en outre :
la réception, par le système de traitement, d'une mesure de pression artérielle externe effectuée à l'aide d'un dispositif de pression artérielle distinct d'un dispositif (101 ; 500) comprenant le capteur radar ;
la comparaison, par le système de traitement, de la mesure de pression artérielle externe et de la mesure de pression artérielle déterminée ; et
la création, par le système de traitement, d'un profil d'étalonnage pour une utilisation afin de modifier une future mesure de pression artérielle déterminée,
dans lequel, éventuellement, un modèle d'apprentissage automatique est modifié sur la base du profil d'étalonnage spécifique à l'utilisateur.

6. Procédé de mesure de la pression artérielle selon la revendication 1, dans lequel le capteur radar et le système de traitement sont intégrés dans un dispositif hub d'assistant domestique qui comprend en outre un écran d'affichage et un haut-parleur, dans lequel l'indication de la pression artérielle déterminée est fournie à l'aide de l'écran d'affichage, du haut-parleur ou des deux.

7. Procédé de mesure de la pression artérielle selon la revendication 1, dans lequel le dispositif de mesure de la pression artérielle sans contact est configuré pour être stationnaire sur une surface (730 ; 830).

8. Système de mesure de la pression artérielle (100 ; 200 ; 300), comprenant :
un dispositif de mesure de la pression artérielle sans contact (101 ; 500) ;
un sous-système radar (120 ; 205) inclus dans le dispositif de mesure de la pression artérielle sans contact, comprenant :
un émetteur radiofréquence (RF) (206) qui émet des signaux RF ; un récepteur RF (207) qui reçoit des signaux de réflexion RF sur la base des signaux RF émis étant réfléchis ; et
un système de traitement (110), comprenant un ou plusieurs processeurs, en communication avec le sous-système radar, dans lequel le système de traitement est configuré pour :
traiter les signaux RF reçus pour obtenir des mesures de fréquence regroupées par distance,
analyser les signaux de réflexion RF reçus traités à une première plage de distance (702 ; 802) correspondant à une première classe de distance pour identifier un premier temps d'une onde de pression d'impulsion au niveau d'une valve aortique (724 ; 820) d'un utilisateur (720) ;
analyser les signaux de réflexion RF reçus traités à une seconde plage de distance (701 ; 801) différente de la première plage de distance et correspondant à une seconde classe de distance pour identifier un second temps de l'onde de pression d'impulsion au niveau d'une extrémité (722 ; 810) de l'utilisateur ;
déterminer un temps de transmission d'impulsion (PTT) de l'onde de pression d'impulsion de la valve aortique de l'utilisateur jusqu'à l'extrémité de l'utilisateur à l'aide du premier temps et du second temps ;
déterminer une pression artérielle de l'utilisateur sur la base du PTT déterminé ; et
fournir une indication de la pression artérielle déterminée.

9. Système de mesure de la pression artérielle selon la revendication 8, comprenant en outre un boîtier, dans lequel le boîtier contient le sous-système radar et le système de traitement
dans lequel, éventuellement, le boîtier contient en outre : un microphone, un haut-parleur (518) et un affichage électronique, dans lequel l'indication de la pression artérielle déterminée est fournie par le biais de l'affichage électronique.

10. Système de mesure de la pression artérielle selon la revendication 8, dans lequel l'extrémité de l'utilisateur est une ou plusieurs mains de l'utilisateur ou
dans lequel l'extrémité de l'utilisateur est un ou plusieurs pieds de l'utilisateur.

11. Système de mesure de la pression artérielle selon la revendication 8, dans lequel l'analyse des signaux de réflexion RF à la première plage de distance et à la seconde plage de distance est réalisée par un ou plusieurs réseaux neuronaux.

12. Système de mesure de la pression artérielle selon la revendication 8, dans lequel le système de traitement est en outre configuré pour déterminer une fréquence cardiaque sur la base de l'analyse des signaux de réflexion RF, dans lequel la détermination de la pression artérielle de l'utilisateur est en outre sur la base de la fréquence cardiaque.

13. Système de mesure de la pression artérielle selon la revendication 8, dans lequel le système de traitement est en outre configuré pour déterminer une amplitude de forme d'onde de pouls dérivée (DPWA) au niveau de l'extrémité de l'utilisateur sur la base de l'analyse des signaux de réflexion RF, dans lequel la détermination de la pression artérielle de l'utilisateur est en outre sur la base de la DPWA.

14. Système de mesure de la pression artérielle selon la revendication 8, dans lequel le système de traitement est en outre configuré pour :
recevoir une mesure de pression artérielle externe effectuée à l'aide d'un dispositif de pression artérielle distinct d'un dispositif comprenant le sous-système radar ;
comparer la mesure de pression artérielle externe et la mesure de pression artérielle déterminée ; et
créer un profil d'étalonnage pour une utilisation afin de modifier une future mesure de pression artérielle déterminée, dans lequel, éventuellement, un modèle d'apprentissage automatique est modifié sur la base du profil d'étalonnage spécifique à l'utilisateur.

15. Support non transitoire lisible par processeur, comprenant des instructions lisibles par processeur configurées pour amener un ou plusieurs processeurs à :
traiter des signaux de réflexion radiofréquence (RF) pour obtenir des mesures de fréquence regroupées par distance, dans lequel les signaux de réflexion RF sont reçus à partir d'un capteur radar (120 ; 205) inclus dans un dispositif de mesure de la pression artérielle sans contact (101 ; 500) ;
analyser les signaux de réflexion radiofréquence (RF) à une première plage de distance (702 ; 801) correspondant à une première classe de distance pour identifier un premier temps d'une onde de pression d'impulsion au niveau d'une valve aortique (724 ; 810) d'un utilisateur (720) ;
analyser les signaux de réflexion RF traités à une seconde plage de distance (702 ; 801) différente de la première plage de distance et correspondant à une seconde classe de distance pour identifier un second temps de l'onde de pression d'impulsion au niveau d'une extrémité (710 ; 810) de l'utilisateur ;
déterminer un temps de transmission d'impulsion (PTT) de l'onde de pression d'impulsion de la valve aortique de l'utilisateur jusqu'à l'extrémité de l'utilisateur à l'aide du premier temps et du second temps ;
déterminer une pression artérielle de l'utilisateur sur la base du PTT déterminé ; et fournir une indication de la pression artérielle déterminée.
